# EUROPEAN PATENT APPLICATION

(11) **EP 1 550 714 A1**
(43) Date of publication of application: **06.07.2005**
(21) Application number: 03733137.8
(22) Date of filing: 28.05.2003
(51) Int. Cl.: C12N 1/20, C12P 7/06

(54) **NOVEL ETHANOL PRODUCING BACTERIUM AND PROCESS FOR PRODUCING ETHANOL**

(30) Priority: 29.05.2002 JP 2002155085
(71) Applicant: COSMO OIL CO., LTD, Minato-ku, Tokyo 105-8528 (JP)
(72) Inventor: NISHIO, Naomichi, Higashi-hiroshima-shi, Hiroshima 739-014 (JP); NAKASHIMADA, Yutaka, Higashi-hiroshima-shi, Hiroshima 739-002 (JP); WATANABE, Shigeyuki, Cosmo Oil Co., Ltd., Satte-shi, Saitama 340-0193 (JP); OTSUKA, Hiroaki, Cosmo Oil Co., Ltd., Satte-shi, Saitama 340-0193 (JP); CHIYODA, Osamu, Cosmo Oil Co., Ltd., Satte-shi, Saitama 340-0193 (JP); TANAKA, Toru, Cosmo Oil Co., Ltd., Tokyo 105-8528 (JP)
(74) Representative: HOFFMANN - EITLE
(86) International application number: PCT/JP2003/006690
(87) International publication number: WO 2003/100037

(57) **Abstract**

A bacterium capable of producing ethanol at a temperature of 40°C or more from a carbon compound which is gaseous at ordinary temperature and ordinary pressure as the raw material, and a process for producing ethanol by culturing the bacterium at 40°C or more.

## Description

### Technical Field

The present invention relates to a bacterium capable of producing ethanol at a high temperature from a gaseous carbon compound such as carbon dioxide as the raw material, and a process for efficiently producing ethanol from the same.

### Background of the Invention

Since there is a background that the exhaustion of petroleum, the carbon dioxide problem accompanied by greenhouse effect and the like, various energy sources have been developed. Particularly, as the movable body energy to be used in transportation and the like, development of a novel liquid fuel considered to be relatively easily introduced in view of the use of existing facilities and safety is drawing attention. Examples of the liquid fuel production processes so far developed include production of a hydrocarbon from natural gas, production of methanol from synthetic gas, liquefaction of coal, production of ethanol from molasses and starch. Among these, since octane number is high, ethanol is used as a gasoline base material in foreign countries, and its application is expected also in Japan.

The ethanol production so far employed is its fermentation production which uses microorganisms, and field crops such as sugar cane and corn are used as the raw material. Although it is necessary to produce ethanol at a moderate price when used as a fuel, inexpensive ethanol production is considerably difficult to carry out by the conventional production process, since the raw material competes with food and the raw material becomes expensive. Thus, development of ethanol production techniques which use a cheap raw material has been desired in recent years. One of them is a process in which cheap organic waste such as an agricultural waste and waste woodare used as the raw material and subjected to a saccharification treatment by an acid or enzyme, and ethanol is produced by fermenting the solution. Although production of ethanol at a moderate price can be expected by this process, since the raw material is a waste, there are many problems to be solved in view of cost and techniques, such as the requirement of secondary treatment facilities for the waste water treatment of treated water generated at the saccharification step and high load water after the culturing, so that the development of an ethanol production technique which using no sugar as the raw material has been desired.

As a countermeasure, development of an ethanol production technique using gas such as carbon monoxide and carbon dioxide as the raw material has been proceeded. In this technique, ethanol is produced from a gas obtained by burning or the like as the main material, using a microorganism which can use the gas. *Clostridium ljyungdahlii* (USP-5173429 (1992)) and *Clostridium autoethanogenum* sp. (Jamal Abrini *et al., Arch. Microbiol.,* 161: 345-351 (1994)) are so far known as microorganisms which produce ethanol from a gas as the raw material. It is reported in each case that growth and ethanol production are carried out at 37°C from a carbon monoxide-containing gas as the raw material.

However, when ethanol is produced by using the microorganisms, gas such as carbon monoxide and carbon dioxide to be used as the raw material is produced at high temperature and high pressure conditions in most cases. Accordingly, when a bacterium which grows and produces ethanol at 37°C is used, gas-cooling facilities such as a heat exchanger and a large tank are required, so that a huge cost is required in the facility point of view. Also, since the produced ethanol is toxic for the bacterium, its continuous production by culturing while taking out it from the culture is considered to be an efficient production process, but the bacteria used so far have a problem that large energy is consumed for taking out the product because of the low culturing temperature and ethanol production temperature, so that the low production temperature is disadvantageous for the industrial production application. Accordingly, a bacterium which produces ethanol at higher temperature from a gas as the raw material has been expected.

In addition, while a carbon monoxide-containing gas is used as the raw material in the conventional techniques as described in the above, a green house gas reducing effect can be expected in case that it is possible to obtain a bacterium which can produce ethanol from carbon dioxide as the raw material which is one of the green house gases.

### Disclosure of the Invention

An object of the present invention is to provide a bacterium which can produce ethanol in high efficiency at a higher temperature from gas such as carbon dioxide as the raw material.

Another object of the present invention is to provide a process for efficiently producing ethanol using the bacterium at a high temperature from a gas as the raw material.

The present invention relates to the following (1) to (17).
(1) A bacterium capable of producing ethanol at a temperature of 40°C or more from a carbon compound which is gaseous at ordinary temperature and ordinary pressure as the raw material.
(2) The bacterium according to (1), wherein the carbon compound is at least one selected from the group consisting of carbon monoxide, carbon dioxide, methane, ethane and ethylene.
(3) The bacterium according to (1) or (2), wherein the carbon compound is at least one selected from the group consisting of carbon monoxide and carbon dioxide.
(4) The bacterium according to any one of (1) to (3), which belongs to the genus *Clostridium* or a derivative genus thereof.
(5) The bacterium according to (4), wherein the genus *Clostridium* or a derivative genus thereof is the genus *Clostridium*, the genus *Thermoanaerobacterium*, the genus *Thermoanaerobacter* or the genus *Moorella*.
(6) The bacterium according to any one of (1) to (5), which belongs to the genus *Clostridium*.
(7) The bacterium according to any one of (1) to (6), which is *Clostridium* sp. No. 16-1 (FERM BP-8372), *Clostridium* sp. No. 16-2 (FERM BP-8373), *Clostridium* sp. No. 22-1 (FERM BP-8374) or an analogous bacterium thereof.
(8) A process for producing ethanol, which comprises culturing, at a temperature of 40°C or more, a bacterium capable of producing ethanol at a temperature of 40°C or more from a carbon compound which is gaseous at ordinary temperature and ordinary pressure as the raw material.
(9) The process according to (8), wherein said culturing is carried out in the presence of a carbon compound which is gaseous at ordinary temperature and ordinary pressure.
(10) The process according to (8) or (9), wherein the carbon compound is at least one selected from the group consisting of carbon monoxide, carbon dioxide, methane, ethane and ethylene.
(11) The process according to any one of (8) to (10), wherein the carbon compound is at least one selected from the group consisting of carbon monoxide and carbon dioxide.
(12) The process according to any one of (8) to (11), wherein the bacterium belongs to the genus *Clostridium* or a derivative genus thereof.
(13) The process according to (12), wherein the genus *Clostridium* or a derivative genus thereof is the genus *Clostridium*, the genus *Thermoanaerobacterium*, the genus *Thermoanaerobacter* or the genus *Moorella*.
(14) The process according to any one of (8) to (13), wherein the bacterium belongs to the genus *Clostridium*.
(15) The process according to any one of (8) to (14), wherein the bacterium is *Clostridium* sp. No. 16-1 (FERM BP-8372), *Clostridium* sp. No. 16-2 (FERM BP-8373), *Clostridium* sp. No. 22-1 (FERM BP-8374) or an analogous bacterium thereof.
(16) The process according to any one of (8) to (15), which further comprises:
   vaporizing the produced ethanol to thereby separate it from the culture; and
   liquefying the separated ethanol.
(17) A process for producing ethanol, which comprises:
   culturing, at a temperature of 40°C or more, a bacterium capable of producing ethanol at a temperature of 40°C or more from a carbon compound which is gaseous at ordinary temperature and ordinary pressure as the raw material;
   vaporizing the produced ethanol to thereby separating it from the culture, and
   liquefying the separated ethanol.

### Brief Description of the Drawings

Fig. 1 is a drawing showing ethanol productivity of the bacterial strain of the present invention.
Fig. 2 is a drawing showing ethanol productivity of the bacterial strain of the present invention by enrichment culture.
Fig. 3 is a drawing showing an outline of the continuous culturing system of the present invention.
Fig. 4 is a drawing showing changes of the ethanol production by the continuous culturing of the present invention.

### Best Mode for Carrying Out the Invention

With the object of solving the aforementioned problems, the inventors of the present invention have conducted intensive studies and found as a result that a specified bacterium produces ethanol at a high temperature of 40°C or more from gas such as carbon dioxide as the raw material, and that production of ethanol while continuously recovering the formed ethanol from the culture is possible by the use of such bacterium in an ethanol production process which uses a gas as the raw material at a high temperature, and established the present invention.

The bacterium to be used in the ethanol production process of the present invention is a bacterium capable of producing ethanol at a temperature of 40°C or more (to be referred also to as "ethanol producing bacterium of the present invention" hereinafter). Completely nothing has not been known so far about a bacterium capable of producing ethanol at a high temperature of 40°C or more. Ethanol production temperature of the ethanol producing bacterium of the present invention is preferably from 50 to 100°C, more preferably from 50 to 80°C.

The ethanol producing bacterium of the present invention is preferably the one capable of producing ethanol from a carbon compound as the raw material which is gaseous (also called a gas) at ordinary temperature and ordinary pressure. The term ordinary temperature and ordinary pressure as used herein means 25°C and atmospheric pressure. The carbon compound includes such as carbon monoxide, carbon dioxide, methane, ethane and ethylene, and among these, one or two selected from carbon monoxide and carbon dioxide are preferred. In addition to these carbon compounds, hydrogen, hydrogen sulfide and the like can also be used together. Examples of the mixed gas include a mixed gas of carbon monoxide and hydrogen, a mixed gas of carbon dioxide and hydrogen, a mixed gas of carbon monoxide, carbon dioxide and hydrogen and the like. In that case other kind of gasses such as nitrogen and the like inert gasses, methane gas and hydrogen sulfide are mixed, they have no particular problems unless the ethanol production is inhibited. Also, mixing ratio of respective components in the mixed gas is not particularly limited, unless the ethanol production is inhibited. In addition, although it is possible to add these carbon compounds in a gaseous state, carbon dioxide may be generated in a culture by adding substance which can be used as the carbon dioxide such as a carbonate generating source. Among these combinations, the use of carbon dioxide and hydrogen has the highest green house gas reducing effect. In this connection, the carbon dioxide and carbon monoxide according to the present invention include those which are produced by oxidation such as burning of a carbon-containing material, namely gasses such as methane and ethane, organic material such as coal and wood. In addition, the hydrogen according to the present invention includes those which are produced by oxidizing hydrogencontaining materials such as hydrogen sulfide or methane, as a matter of course.

The ethanol producing bacterium of the present invention is not particularly limited, with the proviso that it is a bacterium capable of producing ethanol at a high temperature condition of 40°C or more from a carbon compound as the raw material which is gaseous at ordinary temperature and ordinary pressure, and although it may be either an aerobic bacterium or an anaerobic bacterium, its preferred examples include bacteria belonging to the genus *Clostridium* or its derivative genus *Thermoanaerobacterium*, genus *Thermoanaerobacter* and genus *Moorella,* which are classified into CLUSTERS V, VI and VII by the report of Collins *et al*. (Collins M.D., *International Journal of Systematic Bacteriology,* Oct., 812-826 (1994)). In addition, *Clostridium* sp. No. 16-1, *Clostridium* sp. No. 16-2 and *Clostridium* sp. No. 22-1, which are bacteria of new species found for the first time by the present invention, and analogous bacteria having similar properties to the bacteria as the species can also be used suitably. *Clostridium* sp. No. 16-1, *Clostridium* sp. No. 16-2 and *Clostridium* sp. No. 22-1 have been deposited on May 2, 2003, as FERM BP-8372, FERM BP-8373 and FERM BP-8374, respectively in International Patent Organism Depositary, National Institute of Advanced Industrial Science and Technology (Central 6, 1-1 Higashi, Tsukuba, Ibaraki, Japan). In this connection, as described in the above, the bacteria found by the present invention are bacteria of new species belonging to the genus *Clostridium*, and although they also have the properties of the genus *Thermoanaerobacterium*, genus *Thermoanaerobacter* and genus *Moorella* which are derivative species of the genus *Clostridium*, their various properties as the index in determining the genus and species are clearly different from known bacteria of the same genus, so that a possibility of being new species is remarkably high. In this connection, the bacteria found by the present invention also produce organic acids such as acetic acid in addition to ethanol.

In addition, mutants of the ethanol producing bacterium of the present invention are included within the scope of the present invention as analogous bacterium, so far as said mutants strain produce ethanol at a high temperature condition of 40°C or more. Examples of the treatment for inducing mutation include ultraviolet ray irradiation, X-ray irradiation, radiation exposure, treatment with mutagen such as nitrosoguanidine and acridine dye, or treatment by genetic engineering techniques and the like.

According to the culturing process using an anaerobic bacterium, although it is principally the same as the case of general microorganisms, it is necessary to prevent contamination of oxygen, so that a process to stand still or shake in a culture vessel sealed with a butyl rubber stopper or the like is used in a laboratory. When the scale is relatively large, a generally used fermentor can be used, and it is possible to make an anaerobic condition by replacing oxygen in the apparatus with inert gas such as nitrogen or raw material gas such as carbon dioxide.

In addition to gaseous carbon compounds such as carbon dioxide and carbon monoxide, inorganic salts which can generate carbon dioxide such as carbonatescan also be used as the carbon sources to be used in the culturing. However, the carbon sources are not limited to gasses, and raw materials generally used in the culturing such as saccharides and amino acids can also be used together. In this connection, not only the ethanol producing bacterium of the present invention can produce ethanol from a gas as the raw material, but it also can produce ethanol when a sugar is used as the raw material (cf. Example 1).

In addition, there is also no particular limitations too in the gas supplying process, and its examples include a process in which pressurization is carried out in order to dissolve a gas in the medium and a process in which a gas is continuously supplied at ordinary pressure. As a process for dissolving in the medium, it is possible to add salts which can generate gases, such as carbonates and bicarbonates.

As the nitrogen sources, various nitrogen compounds generally useful in fermentation, ammonium salts such as ammonium chloride and nitrates such as sodium nitrate can be used. In addition, components to be added to the medium are not particularly limited too, and it is a process generally used in the culturing to add inorganic compounds such as potassium dihydrogenphosphate, magnesium sulfate, manganese sulfate, sodium chloride, cobalt chloride, calcium chloride, zinc sulfate, copper sulfate and sodium molybdate and vitamin sources such as yeast extract.

The culturing mode is not particularly limited, and in addition to the generally used stirred tank reactor, a single stage or multistage air lift reactor, a dry tube type fermentor, a packing type fermentor, a fluidized bed reactor and the like can also be used.

Ethanol is produced by the ethanol production process of the present invention by culturing at a high temperature condition of 40°C or more from a gas as the raw material and it is found that its continuous production is possible when the ethanol formed by culturing at a high temperature is vaporized and separated from the culture, since the production inhibition by the accumulation of ethanol can be improved and the vaporized ethanol discharged together with the unreacted gas can be easily separated and recovered by a cooling or the like means. In addition to the aforementioned ethanol production which uses a gas as the raw material, when a bacterium capable of forming ethanol at a high temperature condition is used, such a continuous culturing can also be applied to an ethanol production which uses a conventional sugar or the like raw material. In this connection, the medium composition in said continuous culturing is the same as described in the foregoing and has no particular limitation.

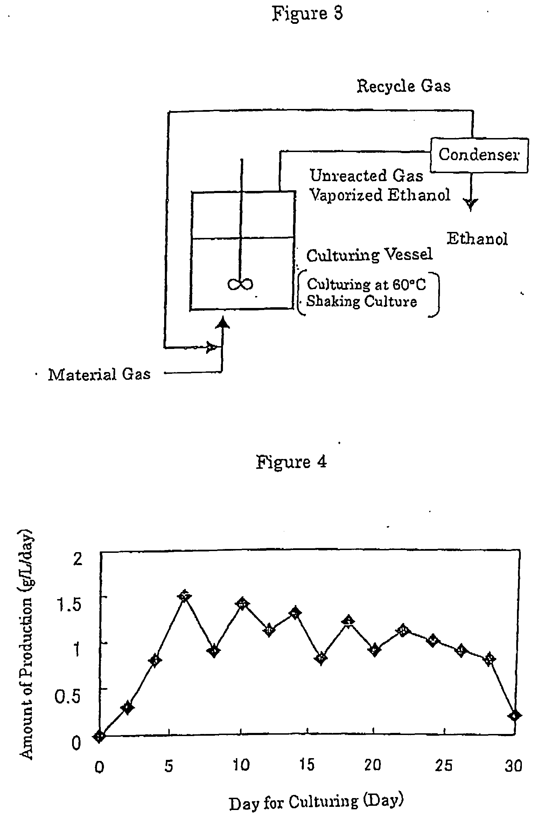

In addition, there is no particular limitation about the culturing mode in said continuous production. Although it is necessary in general to increase cell density at the time of culturing in order to carry out efficient production, the conventional processes well known to those skilled in the art can be used in the present invention, too. For example, in addition to the process shown in the flow chart of Fig. 3, a process which uses concentrated cells or a process which uses immobilized cells can also be used. Examples of the immobilization process include a generally used beads-applied process, a membrane-applied process, a hollow fiber-applied process and the like. In this connection, in the conventional hollow fiber-applied process for the production of ethanol from a sugar as the raw material, technical problem such as clogging due to the raw material has been pointed out, but it is possible to solve this problem by from a gas as the raw material.

Regarding the conditions for vaporizing the formed ethanol, it is possible under any one of the pressurized, ordinary pressure and reduced pressure conditions, but it is desirable to decide by taking into consideration of temperature condition, solubility of the gas and the like factors from the viewpoint of continuous nature of the culturing.

Regarding the method for liquefying vaporized ethanol, there is no particular limitation, and conventionally known cooling methods can be employed, such as a cooling which uses water as the coolant, a cooling which uses air as the coolant and a method which uses a gas as the coolant. In addition, there is no particular limitation regarding the cooling temperature, so long as it is a temperature at which the vaporized ethanol is liquefied, and it is desirable to carry out it at a temperature of the boiling point or less of ethanol (78°C or less) in the case of ordinary pressure.

The present invention is described in the following based on illustrative examples, but the present invention is not restricted by these examples.

### Example 1

Although there are thermophilic acetic acid bacteria in the conventionally known congeneric species or sibling species, it is considered that the bacteria found by the present invention are new bacterial species since they are different from the conventionally known bacteria of the same genus in terms of their ability to produce ethanol and other various properties which are described in the following. Among the found bacterial strains, taxonomic properties of strains No. 16-1, No. 16-2 and No. 22-1 are described. Since formal names are not given yet, they are expressed in the present invention as *Clostridium* sp. No. 16-1 (FERM BP-8372), *Clostridium* sp. No. 16-2 (FERM BP-8373) and *Clostridium* sp. No. 22-1 (FERM BP-8374). Examination of the bacteriological properties was carried out in accordance with the method described in "*Classification and Identification of Microorganisms* (The last volume)" (edited by T. Hasegawa, published by Gakkai Shuppan Center (written in Japanese)) and *Bergey's Manual of Systematic Bacteriology* (1984). In addition, estimation of the belonging taxon was carried out by examining about 500 bp of partial nucleotide sequence of 16S rDNA.

### (1) Taxonomical properties of No. 16-1

### Creation method:

This strain is a bacterial strain isolated from a soil sample collected in Shiobara, Tochigi, Japan. 5ml of the liquid medium shown in Table 1 was dispensed into test tubes, and after sterilization, about 0.5 g.of each soil sample was added. The tube was sealed with a butyl rubber stopper, and then the gas phase was replaced by a gas containing hydrogen (75%) and carbon dioxide (25%), followed by subculture at an interval of 3 weeks at 55°C on a shaker. After carrying out the subculture twice with the liquid medium, single colony was isolated by a roller tube method (*Methods in Microbiology*, Vol.3 B, paragraph 117 (1969), Academic Press) using an agar medium prepared by adding 0.5% fructose and 2% agar, followed by culturing at 55°C on a shaker using the liquid medium shown in Table 1 in which the gas phase was replaced by a gas containing hydrogen (75%) and carbon dioxide (25%) to obtain the strain.

### Microscopic observation:

1. Shape and size of cell: single rod or double rods (curved), 0.5 µm in width, 3.0 to 4.0 µm in length
2. Flagella: exist
3. Spore: exist
4. Gram staining: positive (irregular in later stage of culturing)

### Medium composition:

Exemplified in Table 1.

**Table 1**

| Composition of basal medium (in 1 liter of distilled water) | |
|---|---|
| NH₄Cl | 1.0 g |
| KCl | 0.1 g |
| MgSO₄·7H₂O | 0.2 g |
| NaCl | 0.8 g |
| KH₂PO₄ | 0.1 g |
| CaCl₂·2H₂O | 0.02 g |
| Yeast extract | 1.0 g |
| NaHCO₃ | 2.0 g |
| Mineral solution (1) | 10 ml |
| Vitamin solution (2) | 10 ml |
| Resazurin solution (0.1%) | 1 ml |
| Reducing solution (3) | 10 ml |
| pH | 6.5 |
| | |

(1) Mineral solution (in 1 liter of distilled water)

| | |
|---|---|
| Nitrilotriacetic acid (adjusted to pH 6.0 with KOH) | 2.0 g |
| MnSO₄·H₂O | 1.0 g |
| Fe(SO₄)₂(NH₄)₂·6H₂O | 0.8 g |
| CoCl₂·6H₂O | 0.2 g |
| ZnSO₄·7H₂O | 0.2 g |
| CuCl₂·2H₂O | 0.02 g |
| NiCl₂·6H₂O | 0.02 g |
| Na₂MoO₄·2H₂O | 0.02 g |
| Na₂SeO₄ | 0.02 g |
| Na₂WO₄ | 0.02 g |

(2) Vitamin solution (in 1 liter of distilled water)

| | |
|---|---|
| Biotin | 2.0 mg |
| Folic acid | 2.0 mg |
| Pyridoxine hydrochloride | 10.0 mg |
| Thiamine hydrochloride | 5.0 mg |
| Riboflavin | 5.0 mg |
| Nicotinic acid | 5.0 mg |
| Calcium pantothenate | 5.0 mg |
| Vitamin B₁₂ | 0.1 mg |
| p-Aminobenzoic acid | 5.0 mg |
| Thioctic acid | 5.0 mg |

(3) Reducing solution (in 0.1 liter of distilled water)

| | |
|---|---|
| Cysteine hydrochloride monohydrate | 4.0 g |
| Sodium sulfide nonahydrate | 4.0 g |
| Sodium hydroxide | 0.9 g |

### Growth conditions:

Growth on an agar medium prepared by adding 2% of agar to the composition of Table 1 is as follows.
Shape: circular
Fringe: smooth
Upheaval: slightly raised
Surface: smooth, lustrous
Color tone: cream color

### Physiological properties:

Behavior against oxygen: obligate anaerobic
Growth pH range: optimum pH 7.0, growth pH range 5.0 to 8.0
Growth temperature range: optimum temperature 60°C, growth range 40 to 65°C
Indole production: -
Hydrolysis of gelatin: +
Hydrolysis of aesculin: +
Catalase production: -
Formation of pigment: -
Vitamin requirement: thiamine

### Assimilation of carbon sources and the like:

Tests on biochemical properties were carried out by an API system (bioMerieux France) in accordance with the measuring method of API 20A. In addition, assimilation of carbon sources was verified by carrying out the following test as an additional test.

With 1% of each carbon source, 5 ml of the medium of Table 1 was supplemented and put into a test tube, and the strain was inoculated into the thus prepared aseptic medium, followed by stationary cultures at 60°C for 7 days after replacing the gas phase with sterilized nitrogen gas. Regarding the growth, absorbance at 660 nm was measured using a spectrophotometer (UV 2100 PC, manufactured by Shimadzu). When difference in the absorbance at 660 nm from that of the control which contains no carbon source was less than 0.1, it was judged as "not assimilated", 0.1 or more and less than 0.3 as "slightly assimilated", and 0.3 or more as "assimilated".
"Assimilated"
   D-glucose, D-fructose, galactose, D-xylose, arabinose, trehalose, mannitol, lactose, maltose, salicin, D-cellobiose and D-mannose.
   In addition, it also assimilated carbon dioxide under an environment of the presence of carbon monoxide and hydrogen.
"Slightly assimilated"
   Rhamnose and ribose.
"Not assimilated"
   Sorbitol, glycerin and D-raffinose.

### Products:

With 1% of each carbon source whose assimilation was confirmed, 5 ml of the medium of Table 1 was supplemented and put into a test tube, and the strain was inoculated into a prepared aseptic medium followed by stationary culture at 60°C for 7 days after replacing the gas phase with sterilized nitrogen gas. Production of ethanol and acetic acid was confirmed in all of the carbon sources.

Also, when carbon dioxide was used as the carbon source, 5 ml of the medium of Table 1 was put into a test tube, and the strain was inoculated into a prepared aseptic medium followed by shaking culture at 55°C for 7 days after replacing the gas phase with a sterilized gas of carbon dioxide (25%) and hydrogen (75%). In that case, production of ethanol and acetic acid was confirmed.

### Partial nucleotide sequence of 16S rDNA:

Genomic DNA was extracted from the strain using PrepMan (registered trademark) Method (Applied Biosystems, US). Using the thus extracted genomic DNA as the template, a partial nucleotide sequence of about 500 bp of the 16S rDNA was amplified by PCR, and the nucleotide sequence was subjected to sequencing and used for the analysis. MicroSeq (registered trademark) 500 16S rDNA Bacterial Sequencing Kit (Applied Biosystems, US) was used in the purification and cycle sequencing of the PCR product. With regard to the operations from the genomic DNA extraction to cycle sequencing, they were carried out in accordance with the protocol (P/N 4308132 Rev. A) of Applied Biosystems, and GeneAmp PCR System 9600 (Applied Biosystems, US) was used as the thermal cycler, while ABI PRISM 377 DNA Sequencer (Applied Biosystems, US) was used as the DNA Sequencer.

The partial nucleotide sequence of 16S rDNA of the strain was the nucleotide sequence shown in SEQ ID NO:1.

When homology search of the thus obtained 16S rDNA was carried out for a DNA nucleotide sequence data base (GenBank/EMBL/DDBJ) using BLAST, homology was 98.34% with *Thermoanaerobacterium aotearoense* JW/SL-NZ613T, 97.18% with *Thermoanaerobacterium* sp. C-1, 97.18% with *Thermoanaerobacterium* sp. C38-4, 96.97% with *Clostridium* sp. C-4 and 95.79% with *Clostridium* sp. C41-3, so that it was considered that it belongs to the genus *Clostridium.* Comparison with conventional analogous species and the like:

Based on the aforementioned bacteriological properties, No. 16-1 is a strain of obligate anaerobic spore forming rod which is characterized by the production of ethanol and acetic acid as the main fermentation products from carbon dioxide and hydrogen. When these properties are searched with reference to *Bergey's Manual of Determinative Bacteriology,* 8th edition, *Bergey's Manual of Systematic Bacteriology* and *Classification and Identification of Microorganisms* (the last volume), the strain is considered to be a strain belonging to the genus *Clostridium.* In addition, there are no descriptions in *Bergey's Manual of Determinative Bacteriology,* 8th edition, *Bergey's Manual of Systematic Bacteriology,* and *Handbook of Extreme Environmental Microorganisms* (T. Oshima, Science Forum 1991), of bacterial species whose various properties coincide with those of No. 16-1. As bacteria which grow at a high temperature condition of 50°C or more and produce acetic acid by growing on carbon dioxide and hydrogen, *Moorella thermoacetica* (*Clostridium thermoaceticum*), *Moorella thermoautotrophica* (*Clostridium thermoautotrophicum*) and *Acetogenium kivui* are known, but bacteria which produce ethanol and acetic acid from the same materials have not been found yet. In addition, when its properties were compared with those of the bacteria, they were different in terms of the points shown in Table 2.

Since this strain was considered to be a new species belonging to the genus *Clostridium* based on the above results, this was named *Clostridium* sp. No. 16-1.

### (2) Taxonomical properties of No. 16-2

### Creation method:

This strain is a bacterial strain isolated from a soil sample collected in Shiobara, Tochigi, Japan. Into test tubes, 5ml of the liquid medium shown in Table 1 was dispensed, and after sterilization, 0.5 g of each soil sample was added. The tube was sealed with a butyl rubber stopper, and then the gas phase was replaced by a gas containing hydrogen (75%) and carbon dioxide (25%), followed by subculture at an interval of 3 weeks at 55°C on a shaker. After carrying out the subculture twice with the liquid medium, single colony was isolated by the roller tube method using an agar medium prepared by adding 0.5% fructose and 2% agar, followed by culturing at 55°C on a shaker using the liquid medium shown in Table 1 in which the gas phase was replaced by a gas containing hydrogen (75%) and carbon dioxide (25%) to obtain the strain.

### Microscopic observation:

1. Shape and size of cell: single rod or double rods (curved), 0.6 to 0.7 µm in width, 3.0 to 5.0 µm in length
2. Flagella: exist
3. Spore: exist
4. Gram staining: positive (irregular in later stage of culturing)

### Medium composition:

Exemplified in Table 1.

### Growth conditions:

Growth on an agar medium prepared by adding 2% of agar to the composition of Table 1 is as follows.
Shape: circular
Fringe: smooth
Upheaval: slightly raised
Surface: smooth, lustrous
Color tone: cream color

### Physiological properties:

Behavior against oxygen: obligate anaerobic
Growth pH range: optimum pH 7.0, growth pH range 5.0 to 8.0
Growth temperature range: optimum temperature 60°C, growth range 45 to 65°C
Indole production: -
Hydrolysis of gelatin: +
Hydrolysis of aesculin: +
Catalase production: -
Formation of pigment: -
Vitamin requirement: no

### Assimilation of carbon sources and the like:

Tests on biochemical properties were carried out by an API system (bioMerieux France) in accordance with the measuring method of API 20A. In addition, assimilation of carbon sources was verified by carrying out the following test as an additional test.

Into a test tube, 5 ml of the medium of Table 1 supplemented with 1% of each carbon source was added, and the strain was inoculated into the thus prepared aseptic medium, followed by stationary culture at 60°C for 7 days after replacing the gas phase with sterilized nitrogen gas. With regard to the growth, absorbance at 660 nm was measured using a spectrophotometer (UV 2100 PC, manufactured by Shimadzu). When difference in the absorbance at 660 nm from that of the control which contains no carbon source was less than 0.1, it was judged as "not assimilated", 0.1 or more and less than 0.3 as "slightly assimilated", and 0.3 or more as "assimilated".
"Assimilated"
   D-glucose, D-fructose, galactose, xylose, arabinose, trehalose, D-mannitol, lactose, maltose, salicin, glycerin, cellobiose, D-mannose and D-raffinose.
   In addition, it also assimilated carbon dioxide under an environment of the presence of carbon monoxide and hydrogen.
"Slightly assimilated"
   Rhamnose.
"Not assimilated"
   Ribose and sorbitol.

### Products:

Into a test tube, 5 ml of the medium of Table 1 supplemented with 1% of each carbon source whose assimilation was confirmed was added, and the strain was inoculated into the prepared aseptic medium, followed by stationary culture at 60°C for 7 days after replacing the gas phase with sterilized nitrogen gas. Production of ethanol and acetic acid was confirmed in each of the carbon sources.

Also, when carbon dioxide was used as the carbon source, 5 ml of the medium of Table 1 was put into a test tube, and the strain was inoculated into the prepared aseptic medium, followed by shaking culture at 55°C for 7 days after replacing the gas phase with a sterilized gas of carbon dioxide (25%) and hydrogen (75%). In that case, production of ethanol and acetic acid was confirmed.

### Partial nucleotide sequence of 16S rDNA:

Genomic DNA was extracted from the strain using PrepMan (registered trademark) Method (Applied Biosystems, US). Using the thus extracted genomic DNA as the template, a nucleotide sequence of about 500 bp of the 16S rDNA was amplified by PCR, and the nucleotide sequence was subjected to sequencing and used in the analysis. MicroSeq (registered trademark) 500 16S rDNA Bacterial Sequencing Kit (Applied Biosystems, US) was used for the purification and cycle sequencing of the PCR product. With regard to the operations from the genomic DNA extraction to cycle sequencing, they were carried out in accordance with the protocol (P/N 4308132 Rev. A) of Applied Biosystems, and GeneAmp PCR System 9600 (Applied Biosystems, US) was used as the thermal cycler, and ABI PRISM 377 DNA Sequencer (Applied Biosystems, US) as the DNA Sequencer.

The partial nucleotide sequence of 16S rDNA of this strain was the nucleotide sequence shown in SEQ ID NO;2.

When homology search of the thus obtained 16S rDNA was carried out for a DNA nucleotide sequence data base (GenBank/EMBL/DDBJ) using BLAST, it was 99.08% with *Moorella thermoautotrophica (Clostridium thermoautotrophicum*) DSM1974, 98.90% with *Moorella* *thermoacetica* (*Clostridium thermoaceticum*) ATCC 39037, and 98.71% with *Moorella thermoacetica (Clostridium thermoaceticum*) ET-5a, so that it was considered that the strain belongs to the genus *Clostridium*.

### Comparison with conventional analogous species and the like:

Based on the aforementioned bacteriological properties, No. 16-2 is a strain of obligate anaerobic rod which is characterized by the production of ethanol and acetic acid as the main fermentation metabolic products from carbon dioxide and hydrogen. When these properties are retrieved with reference to *Bergey's Manual of Determinative Bacteriology,* 8th edition, *Bergey's Manual of Systematic Bacteriology* and *Classification and Identification of Microorganisms* (the last volume), this is considered to be a strain belonging to the genus *Clostridium.* In addition, there are no descriptions in *Bergey's Manual of Determinative Bacteriology,* 8th edition, *Bergey's Manual of Systematic Bacteriology,* and *Handbook of Extreme Environmental Microorganisms* (T. Oshima, Science Forum 1991), on bacterial species whose various properties coincide with those of No. 16-1. As bacteria which grow at a high temperature condition of 50°C or more and produce acetic acid by growing on carbon dioxide and hydrogen, *Moorella thermoacetica* (*Clostridium thermoaceticum*), *Moorella thermoautotrophica* (*Clostridium thermoautotrophicum*) and *Acetogenium kivui* are known. However, there are no reports which states that the bacteria produce ethanol and acetic acid. In addition, when its properties were compared with those of the bacteria, they were different in terms of the points shown in Table 2.

Since this strain was considered to be a new species belonging to the genus *Clostridium* based on the above results, the strain was named *Clostridium* sp. No. 16-2.

### (3) Taxonomical properties of No. 22-1

### Creation method:

This strain is a bacterial strain isolated from a subterranean soil sample collected in Chiba, Japan. That is, 5 ml of the liquid medium shown in Table 1 was dispensed into test tubes, and after sterilization, 0.5 g of each soil sample was added. The tube was sealed with a butyl rubber stopper, and then the gas phase was replaced by a gas containing hydrogen (75%) and carbon dioxide (25%) to carry out subculture at an interval of 3 weeks by culturing at 55°C on a shaker. After carrying out the subculture twice with the liquid medium, single colony was isolated by the roller tube method using an agar medium prepared by adding 0.5% fructose and 2% agar, followed by culturing the strain at 55°C on a shaker using the liquid medium shown in Table 1 in which the gas phase was replaced by a gas containing hydrogen (75%) and carbon dioxide (25%) to obtain the strain.

### Microscopic observation:

1. Shape and size of cell: single rod or double rods, 0.5 to 0.6 µm in width, 2.0 to 3.0 µm in length
2. Flagella: exist
3. Spore: exist
4. Gram staining: positive

### Medium composition:

Exemplified in Table 1.

### Growth conditions:

Growth on an agar medium prepared by adding 2% of agar to the composition of Table 1 is as follows.
Shape: circular
Fringe: smooth
Upheaval: slightly raised
Surface: smooth, lustrous
Color tone: cream color

### Physiological properties:

Behavior against oxygen: obligate anaerobic
Growth pH range: optimum pH 7.0, growth pH range 5.0 to 7.5
Growth temperature range: optimum temperature 60°C, growth range 40 to 75°C
Indole production: -
Hydrolysis of gelatin: +
Hydrolysis of aesculin: +
Catalase production: -
Formation of pigment: -
Vitamin requirement: no

### Assimilation of carbon sources and the like:

Tests on biochemical properties were carried out by an API system (bioMerieux France) in accordance with the measuring method of API 20A. In addition, assimilation of carbon sources was verified by carrying out the following test as an additional test.

In a test tube, 5 ml of the medium of Table 1 supplemented with 1% of each carbon source was added, and the strain was inoculated into the aseptic medium, followed by stationary culture at 60°C for 7 days after replacing the gas phase with sterilized nitrogen gas. With regard to the growth, absorbance at 660 nm was measured using a spectrophotometer (UV 2100 PC, manufactured by Shimadzu). When difference in the absorbance at 660 nm from that of the control containing no carbon source was less than 0.1, it was judged as "not assimilated", 0.1 or more and less than 0.3 as "slightly assimilated", and 0.3 or more as "assimilated".
"Assimilated"
   D-glucose, D-fructose, galactose, xylose, arabinose, trehalose, ribose, lactose, maltose, salicin, D-cellobiose and D-mannose.
   In addition, it also assimilated carbon dioxide under an environment of the presence of carbon monoxide and hydrogen.
"Slightly assimilated"
   Rhamnose.
"Not assimilated"
   Sorbitol, glycerin and raffinose.

### Products:

In a test tube, 5 ml of the medium of Table 1 supplemented with 1% of each carbon source whose assimilation was confirmed was added, and the strain was inoculated into the prepared aseptic medium, followed by stationary culture at 60°C for 7 days after replacing the gas phase with sterilized nitrogen gas. Production of ethanol and acetic acid was confirmed in each of the carbon sources.

Also, when carbon dioxide was used as the carbon source, 5 ml of the medium of Table 1 was added in a test tube, and the strain was inoculated into the prepared aseptic medium, followed by shaking culture at 55°C for 7 days after replacing the gas phase with a sterilized gas of carbon dioxide (25%) and hydrogen (75%). In that case, production of ethanol and acetic acid was confirmed.

### Partial nucleotide sequence of 16S rDNA:

Genomic DNA was extracted from the strain using PrepMan (registered trademark) Method (Applied Biosystems, US). Using the thus extracted genomic DNA as the template, a nucleotide sequence of about 500 bp of the 16S rDNA was amplified by PCR, and the nucleotide sequence was subjected to sequencing and used for the analysis. MicroSeq (registered trademark) 500 16S rDNA Bacterial Sequencing Kit (Applied Biosystems, US) was used in the purification and cycle sequencing of the PCR product. With regard to the operations from the genomic DNA extraction to cycle sequencing, they were carried out in accordance with the protocol (P/N 4308132 Rev. A) of Applied Biosystems, and GeneAmp PCR System 9600 (Applied Biosystems, US) was used as the thermal cycler, and ABI PRISM 377 DNA Sequencer (Applied Biosystems, US) as the DNA Sequencer.

The partial nucleotide sequence of 16S rDNA of the strain was the nucleotide sequence shown in SEQ ID NO;3.

When homology search of the thus obtained 16S rDNA was carried out for a DNA nucleotide sequence data base (GenBank/EMBL/DDBJ) using BLAST, it showed a homology of 98.14% with *Thermoanaerobacterium aotearoense* JW/SL-NZ613T, 97.58% with *Clostridium thermoamylolyticum* DMS2335 and 97.18% with *Thermoanaerobacterium* sp. c38-4, so that it was considered that the strain belongs to the genus *Clostridium*.

### Comparison with conventional analogous species and the like:

Based on the aforementioned bacteriological properties, No. 16-2 is a strain of obligate anaerobic rod which is characterized by the production of ethanol and acetic acid as the main fermentation products from carbon dioxide and hydrogen. When the properties are retrieved with reference to *Bergey's Manual of Determinative Bacteriology,* 8th edition, *Bergey's Manual of Systematic Bacteriology* and *Classification and Identification of Microorganisms* (the last volume), this is considered to be a strain belonging to the genus *Clostridium*. In addition, there is no description in *Bergey's Manual of Determinative Bacteriology,* 8th edition, *Bergey's Manual of Systematic Bacteriology,* and *Handbook of Extreme Environmental Microorganisms* (T. Oshima, Science Forum 1991), on bacterial species whose various properties coincide with those of No. 22-1. As bacteria which grow at a high temperature condition of 50°C or more and produce acetic acid by growing on carbon dioxide and hydrogen, *Moorella thermoacetica* (*Clostridium thermoaceticum*), *Moorella thermoautotrophica* (*Clostridium thermoautotrophicum*) and *Acetogenium kivui* are known, but there are no reports stating that the bacteria produce ethanol and acetic acid (Table 2). Also, although the partial nucleotide sequence of 16S rDNA of the strain was similar to that of No. 16-1, they were distinguishable based on their differences in the cell shape and vitamin requirement. In addition, when its properties were compared with those of the bacteria, they were different in terms of the points shown in Table 2.

Since the strain was considered to be a new species belonging to the genus *Clostridium* based on the above results, it was named *Clostridium* sp. No. 22-1.

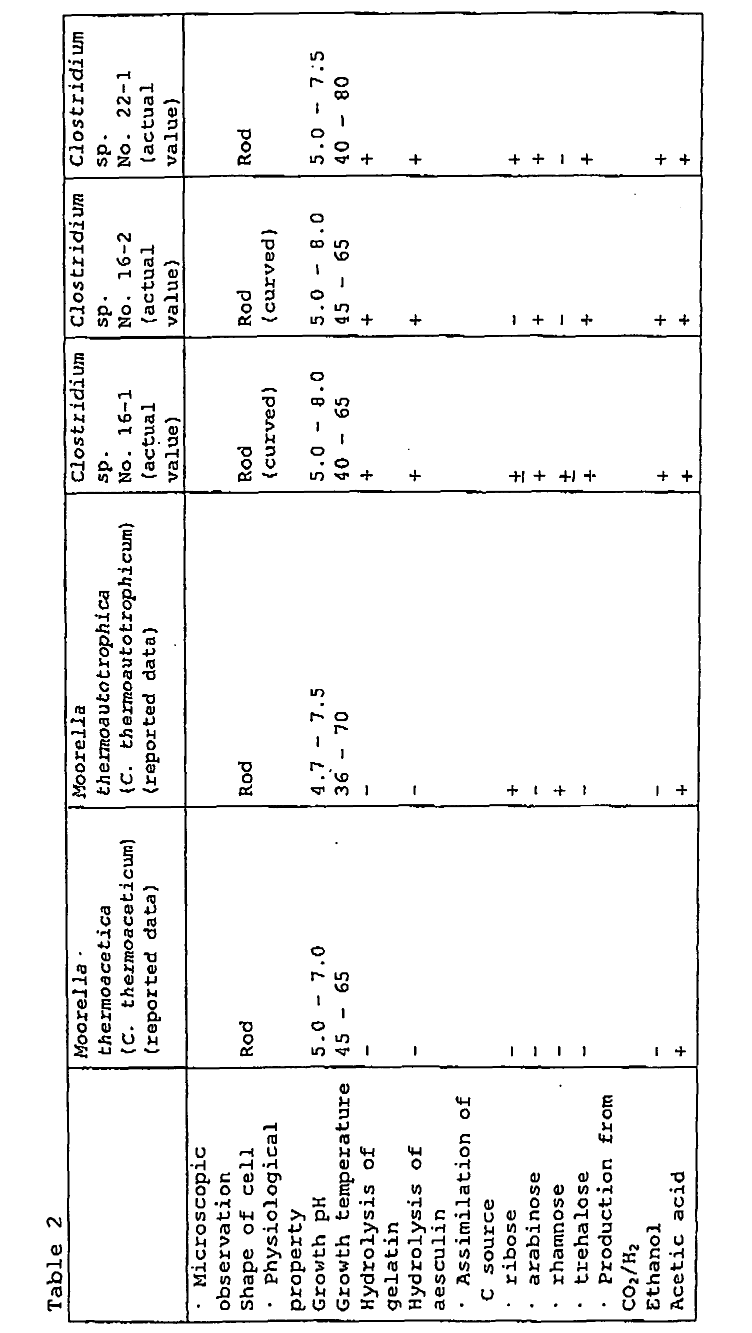

### Example 2

Each of *Clostridium* sp. No. 16-1, *Clostridium* sp. No. 16-2 and *Clostridium* sp. No. 22-1 obtained in the present invention was cultured in the following manner. Into test tubes, 5ml of the medium shown in Table 1 was dispensed and sterilized, and the gas phase was replaced by a sterilized gas containing carbon dioxide (25%) and hydrogen (75%), followed by adjusting to 2 atmospheric pressure with the same gas. 250 µl of the culture broth obtained by culturing each strain using the same medium was added thereto using a sterilized syringe and cultured at 55°C for 10 days with shaking at 150 rpm. A portion of the culture broth was centrifuged to separate cells and the product was determined by gas chromatography. As a result, 0.5 mM of ethanol was produced by *Clostridium* sp. No. 16-1, 1.8 mM of the same by *Clostridium* sp. No. 16-2 and 1.5 mM of the same by *Clostridium* sp. No. 22-1.

### Example 3

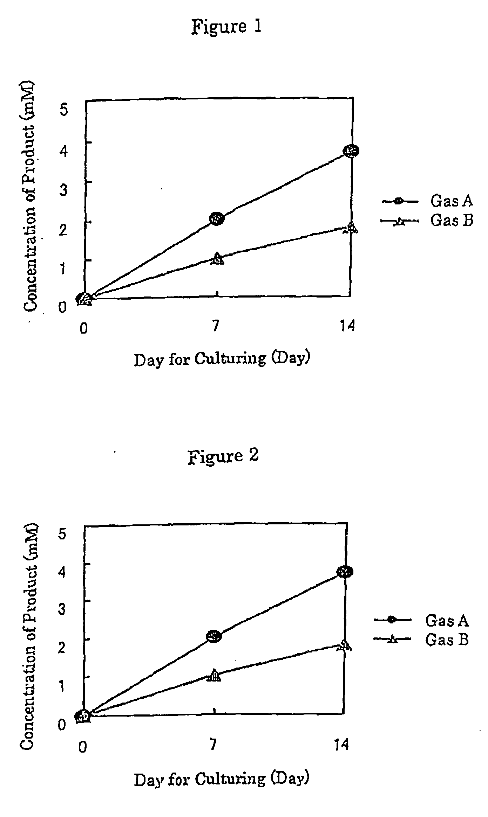

*Clostridium* sp. No. 16-2 was cultured in the following manner. Into test tubes, 5 ml of the medium shown in Table 1 was dispensed. After sterilization, the gas phase was replaced by a sterilized gas containing carbon monoxide (60%), carbon dioxide (10%) and hydrogen (30%) (to be referred to as gas A hereinafter), or a sterilized gas containing carbon dioxide (25%) and hydrogen (75%) (to be referred to as gas B hereinafter) followed by adjusting to 2 atmospheric pressure with each gas. 250 µl of the culture broth obtained by culturing the strain using the same medium was added thereto using a sterilized syringe and cultured at 55°C with shaking at 150 rpm. A portion of the culture broth on the 7th or 14th day was treated with a centrifuge to separate cells and to carry out determination of the product by gas chromatography. As a result, the maximum ethanol production was 3.8 mM when *Clostridium* sp. No. 16-2 was cultured using the gas A (Fig. 1).

### Example 4

An enrichment culturing was carried out using *Clostridium* sp. No. 16-2. Stationary culture was carried out at 60°C for 3 days using a medium prepared by adding 0.5% fructose to the medium shown in Table 1, and the resulting cells were recovered by centrifugation. Into test tubes, 5ml of the medium shown in Table 1 was dispensed. After sterilization, the gas phase was replaced by a sterilized gas containing carbon dioxide (25%) and hydrogen (75%), followed by adjusting to 2 atmospheric pressure with the same gas. The cells adjusted to an absorbance at 660 nm to be 4.0 were added thereto using a sterilized syringe and cultured at 55°C with shaking at 150 rpm. A portion of the culture broth on the 7th or 14th day was centrifuged to separate cells and to carry out determination of the product by gas chromatography. As a result, ethanol production was confirmed at the maximum of 11 mM (Fig. 2).

### Example 5

Continuous culture was carried out using concentrated cells of *Clostridium* sp. No. 16-2. Stationary culture was carried out at 60°C for 5 days using a medium prepared by adding 0.5% fructose to the medium shown in Table 1, and the resulting cells were recovered by centrifugation. Into a 1 liter capacity culture vessel, a 300 ml portion of the medium shown in Table 1 was added and sterilized, and then the gas phase was replaced with a sterilized gas containing carbon dioxide (25%) and hydrogen (75%). The recovered cells were prepared such that the absorbance at 660 nm became 4.0, and added to the culture vessel using a syringe, followed by shaking culture at 60°C and ordinary pressure at 800 rpm using the apparatus shown in Fig. 3. The solution obtained from the outlet of condenser using water of 20°C was recovered, and determination of ethanol was carried out by gas chromatography. As a result of 28 days of the continuous culturing, it was able to maintain an average production of 1.0 g/liter/day (Fig. 4).

The present invention is precisely explained with reference to specific embodiments but it is apparent for those skilled in the art that various changes and modifications can be carried out without departing from the spirit and scope of the present invention.

The present application is based on Japanese Patent Application No.2002-155085 filed on May 29, 2002 and the entire content is incorporated herein by reference.

### Industrial Applicability

According to the present invention, a bacterium which can efficiently produces ethanol from gasses such as carbon dioxide as the raw material at a high temperature of 40°C or more can be provided. In addition, by the use of the bacterium, it is possible that inexpensive, efficient and continuous production of ethanol from gasses such as carbon dioxide as a cause of the global warming, directly without cooling.

## Claims

1. A bacterium capable of producing ethanol at a temperature of 40°C or more from a carbon compound which is gaseous at ordinary temperature and ordinary pressure as the raw material.

2. The bacterium according to claim 1, wherein the carbon compound is at least one selected from the group consisting of carbon monoxide, carbon dioxide, methane, ethane and ethylene.

3. The bacterium according to claim 1 or 2, wherein the carbon compound is at least one selected from the group consisting of carbon monoxide and carbon dioxide.

4. The bacterium according to any one of claims 1 to 3, which belongs to the genus *Clostridium* or a derivative genus thereof.

5. The bacterium according to claim 4, wherein the genus *Clostridium* or a derivative genus thereof is the genus *Clostridium*, the genus *Thermoanaerobacterium*, the genus *Thermoanaerobacter* or the genus *Moorella*.

6. The bacterium according to any one of claims 1 to 5, which belongs to the genus *Clostridium*.

7. The bacterium according to any one of claims 1 to 6, which is *Clostridium* sp. No. 16-1 (FERM BP-8372), *Clostridium* sp. No. 16-2 (FERM BP-8373), *Clostridium* sp. No. 22-1 (FERM BP-8374) or an analogous bacterium thereof.

8. A process for producing ethanol, which comprises culturing, at a temperature of 40°C or more, a bacterium capable of producing ethanol at a temperature of 40°C or more from a carbon compound which is gaseous at ordinary temperature and ordinary pressure as the raw material.

9. The process according to claim 8, wherein said culturing is carried out in the presence of a carbon compound which is gaseous at ordinary temperature and ordinary pressure.

10. The process according to claim 8 or 9, wherein the carbon compound is at least one selected from the group consisting of carbon monoxide, carbon dioxide, methane, ethane and ethylene.

11. The process according to any one of claims 8 to 10, wherein the carbon compound is at least one selected from the group consisting of carbon monoxide and carbon dioxide.

12. The process according to any one of claims 8 to 11, wherein the bacterium belongs to the genus *Clostridium* or a derivative genus thereof.

13. The process according to claim 12, wherein the genus *Clostridium* or a derivative genus thereof is the genus *Clostridium,* the genus *Thermoanaerobacterium*, the genus *Thermoanaerobacter* or the genus *Moorella*.

14. The process according to any one of claims 8 to 13, wherein the bacterium belongs to the genus *Clostridium.*

15. The process according to any one of claims 8 to 14, wherein the bacterium is *Clostridium* sp. No. 16-1 (FERM BP-8372), *Clostridium* sp. No. 16-2 (FERM BP-8373), *Clostridium* sp. No. 22-1 (FERM BP-8374) or an analogous bacterium thereof.

16. The process according to any one of claims 8 to 15, which further comprises:
vaporizing the produced ethanol to thereby separate it from the culture; and
liquefying the separated ethanol.

17. A process for producing ethanol, which comprises:
culturing, at a temperature of 40°C or more, a bacterium capable of producing ethanol at a temperature of 40°C or more from a carbon compound which is gaseous at ordinary temperature and ordinary pressure as the raw material;
vaporizing the produced ethanol to thereby separating it from the culture, and
liquefying the separated ethanol.


SEQUENCE LISTING

<110> COSMO OIL CO., LTD.

<120> Novel ethanol-producing microorganism and process for producing ethanol

<130> P044719

<150> JP 2002-155085
<151> 2002-05-29

<160> 3

<170> PatentIn Ver. 2.1

<210> 1
<211> 492
<212> DNA
<213> Clostridium sp. No. 16-1

<400> 1
gagtttgatc ctggctcagg acgaacgctg gcggcgtgcc taacacatgc aagtcgagcg 60
aagggagtac tacggtacga acttagcggc ggacgggtga gtaacgcgtg gacaatctac 120
cctgtagacy gggataacac cccgaaaggg gtgctaatac cggataatgt caagagtggc 180
atcayttttt gaagaaagga gaaatccgct ataggatgag tccgcgtccc attagctagt 240
tggcggggta aaagcccacc aaggcgacga tgggtagccg gcctgagagg gtgaacggcc 300
acactggaac tgagacacgg tccagactcc tacgggaggc agcagtgggg aatattgtgc 360
aatgggggaa accctgacac agcgacgccg cgtgagcgaa gaaggccttc gggtcgtaaa 420
gctcaatagt atgggaagat aatgacggta ccatacgaaa ccccggctaa ctacgtgcca 480
gcagccgcgg ta 492

<210> 2
<211> 544
<212> DNA
<213> Clostridium sp. No. 16-2

<400> 2
gagtttgatc ctggctcagg acaaacgctg gcggcgtgcc taacacatgc aagtcgagcg 60
gtctttaatt ggggaaatct tcggatggaa ccgattaaag atagcggcgg acgggtgagt 120
aacgcgtggg taatctaccc ttcagactgg gataacaccg ggaaactggt gctaataccg 180
gatacggtct acgggaggca tcttctgtag aagaaaggtg gcgcaagcta ccgctgaagg 240
atgagcccgc gtcccattag ctagttggtg aggtaatggc tcaccaaggc gacgatgggt 300
agccggcctg agagggtggt cggccacact gggactgaga cacggcccag actcctacgg 360
gaggcagcag tggggaatct tgcgcaatgg gcgaaagcct gacgcagcaa cgccgcgtga 420
gcgatgaagg ccttcgggtt gtaaagctct gtcatcaggg acgaagtctt aaaggcgaat 480

agtctttaag gtgacggtac ctgaggagga acccccggct aactacgtgc cagcagccgc 540
ggta 544

<210> 3
<211> 494
<212> DNA
<213> Clostridium sp. No. 22-1

<400> 3
gagtttgatc ctggctcagg acgaacgctg gcggcgtgcc taacacatgc aagtcgagcg 60
aagggagtac tacggtacga acttagcggc ggacgggtga gtaacgcgtg gacaatctac 120
cctgtagacy gggataacac cccgaaaggg gtgctaatac cggataatgt caagaggtgg 180
catcayytyt tgaagaaagg agaaatccgc tataggatga gtccgcgtcc cattagctag 240
ttggcggggt aaaagcccac caaggcgacg atgggtagcc ggcctgagag ggtgaacggc 300
cacactggaa ctgagacacg gtccagactc ctacgggagg cagcagtggg gaatattgtg 360
caatggggga aaccctgaca cagcgacgcc gcgtgagcga agaaggcctt cgggtcgtaa 420
agctcaatag tatgggaaga taatgacggt accatacgaa agccccggct aactacgtgc 480
cagcagccgc ggta 494